# DEMANDE DE BREVET EUROPEEN

(11) **EP 0 900 564 A1**
(43) Date de publication de la demande: **10.03.1999**
(21) Numéro de dépôt: 98401885.3
(22) Date de dépôt: 24.07.1998
(51) Int. Cl.: A61K 9/20, A61K 9/00, A61K 31/155

(54) **Comprimé antiseptique vaginal à action prolongée**

(30) Priorité: 04.08.1997 FR 9709944
(71) Demandeur: Institut Pharmaceutique de Recherche, Application et Développement IPRAD, 75010 Paris (FR)
(72) Inventeur: Ponchel, Gilles, 75012 Paris (FR); Desmolin, Henri, 37750 Saint-Avertin (FR)
(74) Mandataire: Sauvage, Renée

(57) **Abrégé**

La composition selon l'invention renferme :
- de 85 à 55 % en poids d'un diluant hydrosoluble choisi parmi le lactose, le mannitol et le sorbitol ;
- de 10 à 30% en poids d'hydroxyéthyl cellulose ;
- de 1 à 15% en poids de polyvinylpyrrolidone ; et
- environ 1% en poids d'un lubrifiant tel que le stéarate de magnésium.

L'invention concerne également un comprimé susceptible de former un gel, fabriqué par compression directe à partir de ladite composition et d'une poudre calibrée, comprenant au moins un principe actif en une quantité pharmaceutiquement active, tel que du digluconate de chlorhexidine.

Le comprimé selon l'invention est utilisable comme antiseptique vaginal à action prolongée.

## Description

La présente invention concerne une composition pharmaceutique, un comprimé susceptible d'être obtenu à partir de ladite composition et qui, en milieu aqueux in vivo, se transforme, par absorption et gonflement, en un gel souple à propriété matricielle, et un procédé de fabrication de ce comprimé.

L'invention concerne en particulier un comprimé utilisable pour une administration vaginale de principes actifs, notamment d'un antiseptique.

On connaît des anesthésiques buccaux à base de lidocaïne et d'excipients, sous la forme de comprimés susceptibles de gonfler pour former un gel muco-adhésif ("Buccal/Gingival Drug Delivery Systems" par NAGAI, Journal of Controlled Release, 6 (1987) 353-360). Chacun des comprimés est formé, d'une part, d'un noyau contenant le principe actif et, d'autre part, d'un revêtement périphérique qui diffère d'une face à l'autre du comprimé, de sorte que celui-ci peut adhérer à la gencive mais pas à la muqueuse de la joue en vis-à-vis.

Ce type de comprimés, qui nécessitent d'être convenablement orientés pour les faire adhérer à la muqueuse voulue, n'est évidemment pas adapté à une administration par voie orale, vaginale ou rectale, par exemple.

Parmi les formes pharmaceutiques vaginales, des comprimés muco-adhésifs ont également été développés, afin de pallier les inconvénients des formes classiques, telles que les ovules, crèmes, gels et solutions, qui ont tendance à s'écouler hors de la cavité vaginale, ce qui aboutit à une réduction de la quantité de principe actif au niveau de la muqueuse et donc en une diminution d'efficacité thérapeutique, ainsi qu'à une sensation de gêne pour la patiente.

On connaît ainsi des formes de type suppositoires à base de miconazole, fabriquées suivant la technologie BIOSERT®, qui peuvent être utilisées, par exemple, comme antifongiques vaginaux. Il s'agit de formes solides à température ambiante, qui sont susceptibles de donner, à la température du corps, une crème bioadhésive à libération prolongée. On comprend toutefois que le mécanisme en question procède par fusion et non par gonflement. Il s'ensuit que ces formes, comme les suppositoires classiques, doivent être conservées à l'abri de la chaleur pour éviter qu'elles ne fondent avant utilisation.

Ces dernières années, des recherches ont été menées afin de proposer d'autres comprimés muco-adhésifs intravaginaux incorporant des systèmes polymériques hydrophiles adaptés à s'hydrater et à gonfler au contact des sécrétions vaginales pour former progressivement un gel muco-adhésif. Le temps de contact des comprimés avec la muqueuse vaginale, et le profil de libération du principe actif, peuvent être maîtrisés par un choix judicieux de leurs excipients. De plus, le gel formé n'a pas tendance à s'écouler hors de la cavité vaginale, ce qui augmente le confort pour la patiente.

Une étude portant sur l'administration à la vache de comprimés à base de poly(acide acrylique) et de carboxyméthylcellulose sodique, formant un gel muco-adhésif, a ainsi été publiée par Gürsoy et al (Bioahesive controlled release system for vaginal delivery, STP Pharma, 1989, 5, 886-892). Une autre étude portant sur l'administration à la femme ménopausée de comprimés bioadhésifs contenant un dérivé d'estriol a été publiée par Eriksen et al (Low dose 17β-estradiol vaginal tablets in the treatment of atrophic vaginitis : a double-blind placebo controlled study, Eur. J. Obstet. Gynecol. Reprod. Biol., 1992, 44(2), 137-144). Voir également Bouckaert et al, The treatment of bacterial vaginosis with a bioadhesive vaginal slow-release tablet with metranidazole, Proceed. Intern. Symp. Control. Rel. Bioact. Metr., 1994, 21, Controlled Release Society, Inc.

Toutefois, ces comprimés connus ont pour inconvénient que leur hydratation est lente, voire très lente, en raison de la faible quantité d'humidité -voir plus loin- présente dans la cavité vaginale. Cette faible vitesse d'hydratation est incompatible avec les exigences relatives à l'administration de certains principes actifs, tels que le digluconate de chlorhexidine, qui nécessitent, d'une part, une dissolution rapide dans le gel formé pour atteindre une concentration élevée de principe actif au contact de la muqueuse, quelques heures seulement après l'introduction du comprimé et, d'autre part, le maintien de cette concentration pendant 24 heures au moins dans le vagin. En conséquence, le gel doit se former très rapidement après l'introduction du comprimé, par exemple dès la première heure, et ne pas s'éroder trop vite ensuite pour permettre une libération prolongée du principe actif.

Le but de cette invention est donc de proposer une composition à partir de laquelle il sera possible de fabriquer un comprimé, comprenant ladite composition et au moins un principe actif, susceptible de former un gel au contact d'une muqueuse, sans présenter les inconvénients des comprimés muco-adhésifs connus.

Ce but est atteint selon l'invention en ce sens qu'elle propose une composition pharmaceutique renfermant :
de 85 à 55 % en poids d'un diluant hydrosoluble choisi parmi le lactose, le mannitol et le sorbitol ;
de 10 à 30% en poids d'hydroxyéthyl cellulose ;
de 1 à 15% en poids de polyvinylpyrrolidone ; et
environ 1% en poids d'un lubrifiant.

Selon une forme d'exécution préférée de l'invention, la composition renferme :
79% en poids d'un diluant hydrosoluble choisi parmi le lactose, le mannitol et le sorbitol ;
15% en poids d'hydroxyéthylcellulose ;
5% en poids de polyvinylpyrrolidone ; et
1% en poids de stéarate de magnésium.

L'hydroxyéthyl cellulose, qui est destinée à former la matrice polymère hydrophile des comprimés selon l'invention, est, comme d'autres éthers de cellulose non ioniques, un polymère inerte et non irritant pour les muqueuses. Il a toutefois été démontré par les inventeurs que l'hydroxyéthyl cellulose présentait de meilleures propriétés de gonflement et de gélification que l'hydroxypropyl cellulose et qu'elle permettait, en outre, d'obtenir des gels particulièrement limpides. On sait de plus qu'elle offre une meilleure adhérence que l'hydroxyméthyl cellulose, compte tenu de sa plus grande masse moléculaire.

Parmi les différents types d'hydroxyéthyl celluloses disponibles sur le marché, on préférera utiliser, dans cette invention, une solution aqueuse d'hydroxyéthyl cellulose présentant une viscosité relativement élevée (viscosité Brookfield de l'ordre de 3400-5500 mPa.s à 25°C), qui permet à la fois une importante pénétration du liquide dans la matrice polymère et une libération plus lente du principe actif qui lui sera incorporé.

Une étude réalisée par les inventeurs sur le gonflement de comprimés constitués de 100% d'hydroxyéthyl cellulose, de masses différentes et de même dureté, a montré que la vitesse de gonflement et de gélification des comprimés augmente tandis que la masse de polymère diminue. Par conséquent, la proportion pondérale d'hydroxyéthyl cellulose dans la composition selon l'invention doit être inférieure à 30%. Elle doit toutefois être supérieure à 10% en poids pour que la composition forme un gel convenable.

Il a en outre été découvert que l'adjonction à l'hydroxyéthyl cellulose d'un diluant hydrosoluble permettait d'obtenir une hydratation rapide de la matrice polymère et de générer le plus rapidement possible un gel sur le site à traiter, en particulier dans la cavité vaginale où les sécrétions sont limitées à 1-3 g par 24 heures, ces sécrétions ayant une teneur en eau qui varie entre 90 et 98%. Un tel diluant peut être le lactose, le mannitol ou le sorbitol, par exemple. Le lactose est transformé, par la flore bactérienne, en acide lactique qui maintient l'acidité du vagin et permet d'éviter les infections vaginales. Toutefois, il est incompatible avec les amines, ce qui conduit à l'exclure si le principe actif est aminé. Le mannitol et le sorbitol sont, quant à eux, compatibles avec les amines et ils permettent en outre la compression directe de la composition mélangée au principe actif. Le choix du diluant hydrosoluble dépendra donc de sa compatibilité avec le principe actif qui doit être ajouté à la composition selon l'invention.

Le sorbitol est préféré pour une utilisation conjointe avec le digluconate de chlorhexidine. Les inventeurs ont en effet découvert que ce composé, particulièrement avide d'eau, avait la faculté de se dissoudre et de créer des pores au sein de la matrice polymère, de sorte qu'il facilite la diffusion du liquide et augmente la vitesse de gélification du comprimé qui sera formé à partir de la composition. La vitesse d'hydratation et de gélification est directement liée à la quantité de sorbitol utilisée. Il a en outre été démontré que, même en utilisant des types différents de sorbitol (Neosorb P 100 T et Neosorb P 60 fournis par ROQUETTE FRERES), ayant des granulométries différentes, on obtenait une poudre ayant une bonne coulabilité par la méthode de l'entonnoir normalisé, en utilisant un appareil ERWEKA, et par volumenométrie (indice de Carr compris entre 5 et 18). De plus, ces poudres se prêtaient bien à la compression, en utilisant des poinçons plats de 12 mm et des poinçons oblongs de 8,5 x 16 mm sur une machine alternative FROGERAIS OA fabriquant 57 comprimés à la minute.

La polyvinylpyrrolidone, ou PVP, est un liant sec hydrosoluble qui confère à la composition selon l'invention une résistance mécanique suffisante pour qu'elle puisse être soumise à une compression directe, après introduction d'un ou plusieurs principes actifs. Elle a en outre des propriétés non irritantes pour des applications topiques, qui en font un composé de choix de la composition selon l'invention.

Les inventeurs ont découvert qu'en raison de sa viscosité, la PVP influençait la vitesse d'hydratation et de gonflement de la matrice polymère. Par suite, la quantité d'une PVP de viscosité moyenne utilisée dans la composition selon l'invention ne devra pas dépasser 15% en poids.

Le lubrifiant, qui est nécessaire à la compression, est utilisé pour ses propriétés de glissement, son pouvoir anti-adhérent et sa capacité à réduire les frictions entre les particules de la composition. Il peut s'agir de tout lubrifiant connu de l'homme du métier, tel que le stéarate de magnésium, de zinc, de calcium, d'aluminium, le talc, etc. Le stéarate de magnésium sera préféré pour une utilisation dans la présente invention, dans la mesure où il est facilement disponible sur le marché.

L'invention concerne également un procédé de fabrication d'un comprimé susceptible de former un gel, qui comprend les étapes consistant :
- à mélanger une poudre calibrée comprenant au moins un principe actif en une quantité pharmaceutiquement active avec la composition définie ci-dessus ; et
- à réaliser une compression directe du mélange de poudres ainsi obtenu.

Bien entendu, le procédé selon l'invention pourrait en outre comprendre, entre les deux étapes précédentes, une étape de précompression avec, par exemple, concassage et calibrage du grain sur une grille de 0,5 à 1 mm ; les propriétés des comprimés n'en seraient pas modifiées. Il n'est toutefois pas nécessaire de prévoir une telle étape supplémentaire.

Dans le cas où le principe actif n'est disponible dans le commerce que sous la forme d'une solution aqueuse, la poudre calibrée est obtenue par déshydratation d'une quantité pharmaceutiquement active du principe actif, et calibrage de la forme pulvérulente ainsi obtenue.

Il a été découvert que la déshydratation pouvait être avantageusement réalisée par lyophilisation de la solution aqueuse contenant le principe actif.

La lyophilisation consiste à sécher une solution, maintenue à une température suffisamment basse pour que l'eau qu'elle renferme soit congelée, de manière à éliminer l'eau par sublimation. Le produit lyophilisé a une texture poreuse favorisant la dissolution ultérieure du principe actif. La lyophilisation permet en outre d'éviter une dénaturation des principes actifs thermolabiles. La concentration de la solution doit être choisie de façon, d'une part, à réduire au maximum la durée de la lyophilisation et donc les coûts et, d'autre part, à éviter une cristallisation du produit.

Les conditions de lyophilisation et l'aspect du lyophilisat peuvent être améliorées par ajout au principe actif d'un support inerte, tel que la maltodextrine, le sorbitol ou le mannitol, et/ou d'un excipient de structuration solidifiant le réseau poreux du lyophilisat, tel que la PVP, les dextranes et les gommes. Il est bien évident que le choix de l'excipient inerte et/ou de l'excipient de structuration, et l'opportunité d'inclure de tels excipients, dépendront de la nature du principe actif utilisé dans le procédé selon l'invention.

En variante, la déshydratation pourra être réalisée par nébulisation de la solution aqueuse contenant le principe actif. Cette technique consiste à pulvériser la solution aqueuse de principe actif en un nuage de fines gouttelettes, dirigées sur une surface chauffante ou dans de l'air chauffé, en vue de vaporiser le solvant. Elle peut être substituée, dans certains cas, à la lyophilisation.

En variante encore, la déshydratation de la solution aqueuse de principe actif pourra être effectuée par dessiccation aux micro-ondes.

L'invention trouve donc une application particulièrement intéressante dans la fabrication de comprimés contenant du digluconate de chlorhexidine, qui ne se trouve que sous forme de solution aqueuse. La chlorhexidine ou 1,6-di-(4-chlorophényl-diguanido)hexane constitue un traitement de choix contre les infections vaginales en raison de son activité bactéricide et bactériostatique à large spectre. Elle est non spécifique et agit sur de nombreux germes gram positifs ou négatifs, sur les mycobactéries, les levures, les moisissures et les éléments sporulés. En outre, la chlorhexidine est faiblement toxique, bien tolérée par la muqueuse vaginale, et les réactions allergiques qu'elle provoque sont rares. De plus, sa très faible absorption au niveau de la muqueuse vaginale autorise son utilisation pendant la grossesse. Le digluconate de chlorhexidine est plus stable que la chlorhexidine elle-même.

Dans le cas où l'invention est appliquée à la fabrication de comprimés contenant du digluconate de chlorhexidine, on préférera que la solution de digluconate soit lyophilisée en présence de mannitol. Le digluconate de chlorhexidine et le mannitol, qui représentent généralement ensemble de 10 à 20% du poids du comprimé, sont avantageusement présents en un rapport pondéral compris entre 1:5 et 7:5 et, de préférence, en un rapport pondéral de 1:1, afin que le mannitol n'influence pas, dans une trop large mesure, la cinétique de gonflement des comprimés.

L'invention concerne également un comprimé adapté à former un gel, susceptible d'être obtenu par le procédé décrit ci-dessus et en particulier un tel comprimé contenant du digluconate de chlorhexidine, pour son utilisation comme antiseptique vaginal à action prolongée. Comme on l'a indiqué ci-dessus, lorsqu'il est utilisé comme forme pharmaceutique vaginale, le comprimé selon l'invention gonfle rapidement, après mise en place dans le vagin, par absorption du liquide environnant. Le comprimé peut ainsi tripler sa masse et il devient limpide en 3 à 4 heures par dissolution de ses composants solubles, jusqu'à former un gel souple qui tapisse les parois de la muqueuse sous forme d'un film contenant le principe actif, lequel film est susceptible de libérer le principe actif de façon régulière et prolongée.

Les comprimés selon l'invention pourront être utilisés dans le cadre de traitements locaux ou systémiques et ils pourront avoir des formes et/ou des dimensions différentes qui facilitent leur mise en place par la patiente. Ils pourront également avoir un poids différent, en fonction de la quantité de principe actif incorporée, liée elle-même à la nature du principe actif, et de la quantité de composition selon l'invention, qui conditionne la vitesse de gonflement du comprimé, ainsi que le profil de libération du principe actif. Des essais comparatifs ont été effectués sur des comprimés de même masse, respectivement plats, de 12 mm de diamètre, et oblongs, de 18 x 7 mm et de 18 x 9 mm, et sur des comprimés de masses différentes. Ces essais ont montré que la forme et la taille des comprimés n'affectaient pas sensiblement leur gonflement. Par contre, des composés de masses différentes avaient des profils de gonflement différents compte tenu de leur teneur différente en hydroxyéthyl cellulose. On préférera en général utiliser des comprimés pesant environ 500 mg.

L'invention sera mieux comprise, et ses avantages ressortiront plus clairement, à la lumière de la description détaillée suivante d'un exemple de forme d'exécution non limitative de celle-ci.

### EXEMPLE

### Fabrication des comprimés

Pour la fabrication d'un comprimé de 500 mg, les proportions de matières de départ utilisées sont les suivantes :
Excipient (composition selon l'invention) : 440 mg dont
79% de sorbitol,
15% d'hydroxyéthyl cellulose,
5% de polyvinylpyrrolidone, et
1% de stéarate de magnésium ;
Poudre active : 60 mg dont
30 mg de digluconate de chlorhexidine, et
30 mg de mannitol.

Le sorbitol était une poudre de sorbitol P 60, de granulométrie fine, cristallisée sous forme gamma, fournie par ROQUETTE FRERES. L'hydroxyéthyl cellulose était fournie par AQUALON France sous la référence HHX, qui avait une concentration de 1% en solution aqueuse et une viscosité Brookfield de 3400-5500 mPa.s à 25°C. La polyvinylpyrrolidone était la PVP K 40, également fournie par ROQUETTE FRERES. Le stéarate de magnésium était fourni par PROLABO⁸. Ces composés ont successivement été introduits dans un mélangeur par retournement tournant à 15 tours/mn et mélangés quelques minutes pour former un mélange homogène.

On a ensuite lyophilisé une solution aqueuse à 20% de digluconate de chlorhexidine, fournie par RHONE-POULENC, avec le mannitol obtenu auprès de ROQUETTE FRERES sous la dénomination commerciale Pearlitol 300 DC, dans un lyophilisateur Bioblock Scientific, Christ alpha 1-4. Le lyophilisat, soumis à une spectrophotométrie UV-visible à une longueur d'onde de 250 nm, présentait le même spectre, et la même absorbance, qu'une solution de digluconate de chlorhexidine non lyophilisée. On n'a donc pas observé de dégradation du principe actif.

La composition et la poudre active ont été mélangées puis des comprimés ont été fabriqués de manière analogue aux comprimés placebos ci-dessous.

### Etude du profil de gonflement de comprimés placebos

Des comprimés placebos de 500 mg ont parallèlement été préparés par compression directe sur une machine rotative KILIAN® à poinçons oblongs de forme légèrement bombée, mesurant 18 mm x 9 mm.

Ces comprimés ont été soumis à un essai de gonflement dans 900 ml d'une solution tampon à 37°C renfermant 0,2% molaire d'acide acétique pour 0,2% molaire d'acétate de sodium, sur un appareil à palettes tournantes de la Pharmacopée Européenne réglé à une vitesse d'agitation de 15 tours/mn. Les résultats de cet essai sont rassemblés sur la figure 1 ci-dessous.

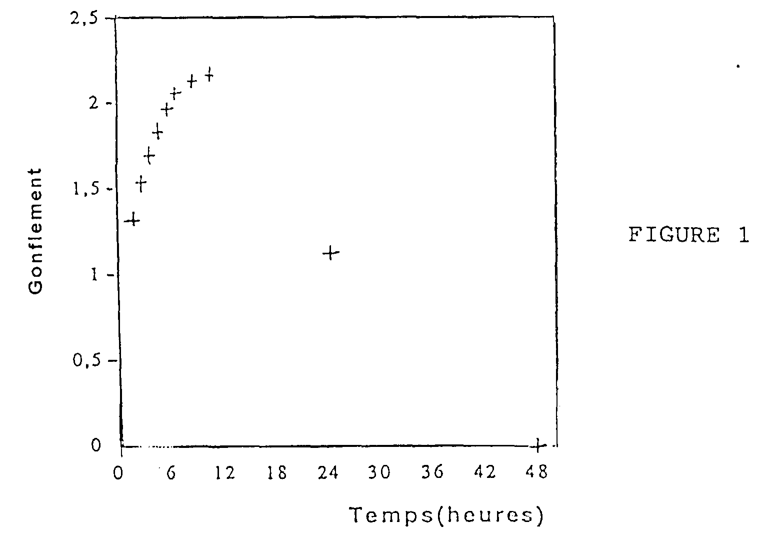

Comme le montre la figure 1, la composition selon cet exemple produit un comprimé qui s'hydrate et gonfle rapidement pour former un gel qui, au bout de 12 heures environ, a atteint un taux de gonflement maximal représentant de 2 à 2,5 fois son volume initial. Le gel subit ensuite une érosion lente et il est complètement dissous au bout de 48 heures.

### Etude de la dissolution du principe actif

Des tests de dissolution ont été effectués sur les comprimés selon l'invention, dans la même solution tampon que celle utilisée ci-dessus. Le profil de libération du principe actif est illustré sur la figure 2.

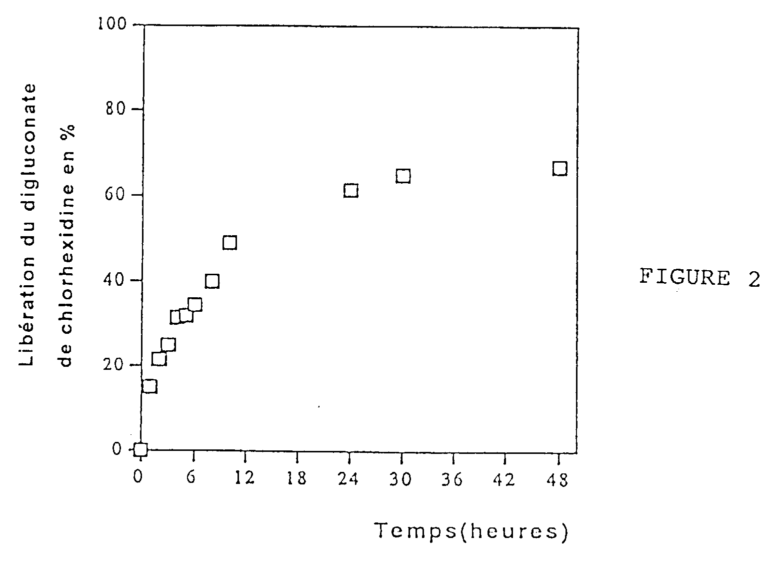

Il ressort de la figure 2 que la composition selon l'invention permet une libération du digluconate de chlorhexidine dès le début du gonflement du comprimé, et qui atteint 30% lorsque tout le comprimé est transformé en gel et 50% après 12 heures. Au bout de 48 heures, la quantité de principe actif solubilisé n'est pas de 100%, du fait que le comprimé gélifié est resté intact ; sa destruction par agitation magnétique à 500 tours/mn aboutit toutefois à une valeur proche de 100%.

Bien que l'invention ait été décrite par référence à un comprimé contenant du digluconate de chlorhexidine, il est bien évident qu'un ou plusieurs autre(s) principe(s) actif(s) pourrai(en)t, en variante, être mélangé(s) de manière analogue à la composition selon l'invention, pour autant qu'ils ne soi(en)t pas présent(s) en une quantité susceptible de modifier de façon significative les propriétés de gonflement du comprimé ainsi formé. Pour une application à des comprimés intravaginaux, ces autres principes actifs peuvent être choisis parmi les anti-herpétiques, les spermicides ou les estrogènes, par exemple.

## Revendications

1. Composition pharmaceutique, renfermant :
de 85 à 55 % en poids d'un diluant hydrosoluble choisi parmi le lactose, le mannitol et le sorbitol ;
de 10 à 30% en poids d'hydroxyéthyl cellulose ;
de 1 à 15% en poids de polyvinylpyrrolidone ; et
environ 1% en poids d'un lubrifiant.

2. Composition selon la revendication 1, caractérisée en ce qu'elle renferme :
79% en poids d'un diluant hydrosoluble choisi parmi le lactose, le mannitol et le sorbitol ;
15% en poids d'hydroxyéthylcellulose ;
5% en poids de polyvinylpyrrolidone ; et
1% en poids de stéarate de magnésium.

3. Procédé de fabrication d'un comprimé susceptible de former un gel, caractérisé en ce qu'il comprend les étapes consistant :
- à mélanger une poudre calibrée, comprenant au moins un principe actif en une quantité pharmaceutiquement active, avec la composition selon la revendication 1 ou 2 ; et
- à réaliser une compression directe du mélange de poudres ainsi obtenu.

4. Procédé selon la revendication 3, caractérisé en ce que ladite poudre calibrée est susceptible d'être obtenue par déshydratation d'une solution aqueuse d'un principe actif en une quantité pharmaceutiquement active, et calibrage de la forme pulvérulente ainsi obtenue.

5. Procédé selon la revendication 4, caractérisé en ce que la déshydratation est réalisée par lyophilisation de la solution aqueuse contenant le principe actif, éventuellement mélangée à un support inerte et/ou à un excipient de structuration.

6. Procédé selon la revendication 5, caractérisé en ce que ledit support inerte est le mannitol.

7. Procédé selon l'une quelconque des revendications 4 à 6, caractérisé en ce que ledit principe actif est le digluconate de chlorhexidine.

8. Procédé selon les revendications 6 et 7, caractérisé en ce que le digluconate de chlorhexidine et le mannitol sont présents en un rapport pondéral compris entre 1:5 et 7:5.

9. Comprimé adapté à former un gel, susceptible d'être obtenu par le procédé selon l'une quelconque des revendications 3 à 8.

10. Comprimé selon la revendication 9, caractérisé en ce qu'il renferme :
de 85 à 55 % en poids de sorbitol ;
de 10 à 30% en poids d'hydroxyéthyl cellulose ;
de 1 à 15% en poids de polyvinylpyrrolidone ;
environ 1% en poids de stéarate de magnésium ; et
de 10 à 20% d'un mélange, lyophilisé et calibré, de digluconate de chlorhexidine et de mannitol en un rapport pondéral compris entre 1:5 et 7:5.

11. Comprimé selon la revendication 10, caractérisé en ce qu'il renferme :
79% en poids de sorbitol ;
15% en poids d'hydroxyéthylcellulose ;
5% en poids de polyvinylpyrrolidone ;
1% en poids de stéarate de magnésium ; et
de 10 à 20% d'un mélange lyophilisé et calibré de digluconate de chlorhexidine et de mannitol en un rapport pondéral de 1:1.

12. Comprimé adapté à former un gel, susceptible d'être obtenu par le procédé selon la revendication 7 ou 8, ou comprimé selon la revendication 10 ou 11, pour son utilisation comme comprimé vaginal.

13. Comprimé adapté à former un gel, susceptible d'être obtenu par le procédé selon la revendication 7 ou 8, ou comprimé selon la revendication 10 ou 11, pour son utilisation comme antiseptique vaginal à action prolongée.
